# EUROPEAN PATENT APPLICATION

(11) **EP 3 594 339 A1**
(43) Date of publication of application: **15.01.2020**
(21) Application number: 18763950.5
(22) Date of filing: 06.03.2018
(51) Int. Cl.: C12N 15/10, C12N 15/63, C12N 9/16

(54) **COMPOSITION CONTAINING C2CL ENDONUCLEASE FOR DIELECTRIC CALIBRATION AND METHOD FOR DIELECTRIC CALIBRATION USING SAME**

(30) Priority: 06.03.2017 KR 20170028567
(71) Applicant: Institute For Basic Science, Daejeon 34126 (KR)
(72) Inventor: KIM, Jin-Soo, Seoul 08831 (KR); KIM, Jung Eun, Seoul 02721 (KR)
(74) Representative: Bates, Philip Ian
(86) International application number: PCT/KR2018/002654
(87) International publication number: WO 2018/164457

(57) **Abstract**

Provided is a dielectric calibration technique using C2cl endonuclease. The dielectric calibration technique is characterized by being especially applicable to eukaryotic cells, for example, to mammalian cells.

<110>	INSTITUTE FOR BASIC SCIENCE

<120>	Composition for Genome Editing Comprising C2c1 Endonuclease and
	Method of Genome Editing Using the Same

<130>	OPP20175581KR

<150>	10-2017-0028567
<151>	2017-03-06

<160>	55

<170>	KopatentIn 3.0

<210>	1
<211>	1129
<212>	PRT
<213>	Artificial Sequence

<220>
<223>	Synthetic (AacC2c1)


<400>	1
Met Ala Val Lys Ser Ile Lys Val Lys Leu Arg Leu Asp Asp Met Pro
 1               5                  10                  15

Glu Ile Arg Ala Gly Leu Trp Lys Leu His Lys Glu Val Asn Ala Gly
            20                  25                  30

Val Arg Tyr Tyr Thr Glu Trp Leu Ser Leu Leu Arg Gln Glu Asn Leu
        35                  40                  45

Tyr Arg Arg Ser Pro Asn Gly Asp Gly Glu Gln Glu Cys Asp Lys Thr
    50                  55                  60

Ala Glu Glu Cys Lys Ala Glu Leu Leu Glu Arg Leu Arg Ala Arg Gln
65                  70                  75                  80

Val Glu Asn Gly His Arg Gly Pro Ala Gly Ser Asp Asp Glu Leu Leu
                85                  90                  95

Gln Leu Ala Arg Gln Leu Tyr Glu Leu Leu Val Pro Gln Ala Ile Gly
            100                 105                 110

Ala Lys Gly Asp Ala Gln Gln Ile Ala Arg Lys Phe Leu Ser Pro Leu
        115                 120                 125

Ala Asp Lys Asp Ala Val Gly Gly Leu Gly Ile Ala Lys Ala Gly Asn
    130                 135                 140

Lys Pro Arg Trp Val Arg Met Arg Glu Ala Gly Glu Pro Gly Trp Glu
145                 150                 155                 160

Glu Glu Lys Glu Lys Ala Glu Thr Arg Lys Ser Ala Asp Arg Thr Ala

1

165                 170                 175

Asp Val Leu Arg Ala Leu Ala Asp Phe Gly Leu Lys Pro Leu Met Arg
            180                 185                 190

Val Tyr Thr Asp Ser Glu Met Ser Ser Val Glu Trp Lys Pro Leu Arg
        195                 200                 205

Lys Gly Gln Ala Val Arg Thr Trp Asp Arg Asp Met Phe Gln Gln Ala
    210                 215                 220

Ile Glu Arg Met Met Ser Trp Glu Ser Trp Asn Gln Arg Val Gly Gln
225                 230                 235                 240

Glu Tyr Ala Lys Leu Val Glu Gln Lys Asn Arg Phe Glu Gln Lys Asn
                245                 250                 255

Phe Val Gly Gln Glu His Leu Val His Leu Val Asn Gln Leu Gln Gln
            260                 265                 270

Asp Met Lys Glu Ala Ser Pro Gly Leu Glu Ser Lys Glu Gln Thr Ala
        275                 280                 285

His Tyr Val Thr Gly Arg Ala Leu Arg Gly Ser Asp Lys Val Phe Glu
    290                 295                 300

Lys Trp Gly Lys Leu Ala Pro Asp Ala Pro Phe Asp Leu Tyr Asp Ala
305                 310                 315                 320

Glu Ile Lys Asn Val Gln Arg Arg Asn Thr Arg Arg Phe Gly Ser His
                325                 330                 335

Asp Leu Phe Ala Lys Leu Ala Glu Pro Glu Tyr Gln Ala Leu Trp Arg
            340                 345                 350

Glu Asp Ala Ser Phe Leu Thr Arg Tyr Ala Val Tyr Asn Ser Ile Leu
        355                 360                 365

Arg Lys Leu Asn His Ala Lys Met Phe Ala Thr Phe Thr Leu Pro Asp
    370                 375                 380

Ala Thr Ala His Pro Ile Trp Thr Arg Phe Asp Lys Leu Gly Gly Asn
385                 390                 395                 400

Leu His Gln Tyr Thr Phe Leu Phe Asn Glu Phe Gly Glu Arg Arg His
                405                 410                 415

Ala Ile Arg Phe His Lys Leu Leu Lys Val Glu Asn Gly Val Ala Arg
            420                 425                 430

Glu Val Asp Asp Val Thr Val Pro Ile Ser Met Ser Glu Gln Leu Asp
        435                 440                 445

Asn Leu Leu Pro Arg Asp Pro Asn Glu Pro Ile Ala Leu Tyr Phe Arg
    450                 455                 460

Asp Tyr Gly Ala Glu Gln His Phe Thr Gly Glu Phe Gly Gly Ala Lys
465                 470                 475                 480

Ile Gln Cys Arg Arg Asp Gln Leu Ala His Met His Arg Arg Arg Gly
                485                 490                 495

Ala Arg Asp Val Tyr Leu Asn Val Ser Val Arg Val Gln Ser Gln Ser
            500                 505                 510

Glu Ala Arg Gly Glu Arg Arg Pro Pro Tyr Ala Ala Val Phe Arg Leu
        515                 520                 525

Val Gly Asp Asn His Arg Ala Phe Val His Phe Asp Lys Leu Ser Asp
    530                 535                 540

Tyr Leu Ala Glu His Pro Asp Asp Gly Lys Leu Gly Ser Glu Gly Leu
545                 550                 555                 560

Leu Ser Gly Leu Arg Val Met Ser Val Asp Leu Gly Leu Arg Thr Ser
                565                 570                 575

Ala Ser Ile Ser Val Phe Arg Val Ala Arg Lys Asp Glu Leu Lys Pro
            580                 585                 590

Asn Ser Lys Gly Arg Val Pro Phe Phe Phe Pro Ile Lys Gly Asn Asp
        595                 600                 605

Asn Leu Val Ala Val His Glu Arg Ser Gln Leu Leu Lys Leu Pro Gly
    610                 615                 620

Glu Thr Glu Ser Lys Asp Leu Arg Ala Ile Arg Glu Glu Arg Gln Arg
625                 630                 635                 640

Thr Leu Arg Gln Leu Arg Thr Gln Leu Ala Tyr Leu Arg Leu Leu Val
                645                 650                 655

Arg Cys Gly Ser Glu Asp Val Gly Arg Arg Glu Arg Ser Trp Ala Lys
            660                 665                 670

Leu Ile Glu Gln Pro Val Asp Ala Ala Asn His Met Thr Pro Asp Trp
        675                 680                 685

Arg Glu Ala Phe Glu Asn Glu Leu Gln Lys Leu Lys Ser Leu His Gly
    690                 695                 700

Ile Cys Ser Asp Lys Glu Trp Met Asp Ala Val Tyr Glu Ser Val Arg
705                 710                 715                 720

Arg Val Trp Arg His Met Gly Lys Gln Val Arg Asp Trp Arg Lys Asp
                725                 730                 735

Val Arg Ser Gly Glu Arg Pro Lys Ile Arg Gly Tyr Ala Lys Asp Val
            740                 745                 750

Val Gly Gly Asn Ser Ile Glu Gln Ile Glu Tyr Leu Glu Arg Gln Tyr
        755                 760                 765

Lys Phe Leu Lys Ser Trp Ser Phe Phe Gly Lys Val Ser Gly Gln Val
    770                 775                 780

Ile Arg Ala Glu Lys Gly Ser Arg Phe Ala Ile Thr Leu Arg Glu His
785                 790                 795                 800

Ile Asp His Ala Lys Glu Asp Arg Leu Lys Lys Leu Ala Asp Arg Ile
                805                 810                 815

Ile Met Glu Ala Leu Gly Tyr Val Tyr Ala Leu Asp Glu Arg Gly Lys
            820                 825                 830

Gly Lys Trp Val Ala Lys Tyr Pro Pro Cys Gln Leu Ile Leu Leu Glu
        835                 840                 845

Glu Leu Ser Glu Tyr Gln Phe Asn Asn Asp Arg Pro Pro Ser Glu Asn
    850                 855                 860

Asn Gln Leu Met Gln Trp Ser His Arg Gly Val Phe Gln Glu Leu Ile
865                 870                 875                 880

Asn Gln Ala Gln Val His Asp Leu Leu Val Gly Thr Met Tyr Ala Ala
                885                 890                 895

Phe Ser Ser Arg Phe Asp Ala Arg Thr Gly Ala Pro Gly Ile Arg Cys
            900                 905                 910

Arg Arg Val Pro Ala Arg Cys Thr Gln Glu His Asn Pro Glu Pro Phe
        915                 920                 925

Pro Trp Trp Leu Asn Lys Phe Val Val Glu His Thr Leu Asp Ala Cys
    930                 935                 940

Pro Leu Arg Ala Asp Asp Leu Ile Pro Thr Gly Glu Gly Glu Ile Phe
945                 950                 955                 960

Val Ser Pro Phe Ser Ala Glu Glu Gly Asp Phe His Gln Ile His Ala
                965                 970                 975

Asp Leu Asn Ala Ala Gln Asn Leu Gln Gln Arg Leu Trp Ser Asp Phe
            980                 985                 990

Asp Ile Ser Gln Ile Arg Leu Arg Cys Asp Trp Gly Glu Val Asp Gly
        995                 1000                1005

Glu Leu Val Leu Ile Pro Arg Leu Thr Gly Lys Arg Thr Ala Asp Ser
    1010                1015                1020

Tyr Ser Asn Lys Val Phe Tyr Thr Asn Thr Gly Val Thr Tyr Tyr Glu
1025                1030                1035                1040

Arg Glu Arg Gly Lys Lys Arg Arg Lys Val Phe Ala Gln Glu Lys Leu

1045                1050                1055

Ser Glu Glu Glu Ala Glu Leu Leu Val Glu Ala Asp Glu Ala Arg Glu
        1060                1065                1070

Lys Ser Val Val Leu Met Arg Asp Pro Ser Gly Ile Ile Asn Arg Gly
    1075                1080                1085

Asn Trp Thr Arg Gln Lys Glu Phe Trp Ser Met Val Asn Gln Arg Ile
  1090                1095                1100

Glu Gly Tyr Leu Val Lys Gln Ile Arg Ser Arg Val Pro Leu Gln Asp
1105                1110                1115                1120

Ser Ala Cys Glu Asn Thr Gly Asp Ile
                1125

<210>    2
<211>    91
<212>    RNA
<213>    Artificial Sequence

<220>
<223>    Synthetic (sgRNA for AacC2c1, wherein each n is independently
         selected from a, u, c, and g)

<400>    2
gucuagagga cagaauuuuu caacgggugu gccaauggcc acuuuccagg uggcaaagcc    60

cguugagcuu cucaaaucug agaaguggnn n                                   91

<210>    3
<211>    91
<212>    RNA
<213>    Artificial Sequence

<220>
<223>    Synthetic (sgRNA for AacC2c1)

<400>    3
gucuagagga cagaauuuuu caacgggugu gccaauggcc acuuuccagg uggcaaagcc    60

cguugagcuu cucaaaucug agaaguggca c                                   91

<210>    4
<211>    7
<212>    PRT
<213>    Artificial Sequence

<220>

<223>    Synthetic (NLS)

<400>    4
Pro Lys Lys Lys Arg Lys Val
  1               5

<210>    5
<211>    60
<212>    DNA
<213>    Artificial Sequence

<220>
<223>    Synthetic (CMV-AacC2c1-CN-1F-Hind3 primer)

<400>    5
gctggctagc gtttaaactt aagcttggta ccgccaccat ggcagtgaaa agcatcaaag    60

                                                                      60

<210>    6
<211>    39
<212>    DNA
<213>    Artificial Sequence

<220>
<223>    Synthetic (CMV-AacC2c1-CN-1R-BamH1 primer)

<400>    6
aacatcgtat gggtaggatc ctcaggcgta gtcgggcac                            39

<210>    7
<211>    242
<212>    DNA
<213>    Artificial Sequence

<220>
<223>    Synthetic (Aac-sgRNA cloning vector)

<400>    7
ggtctagagg acagaatttt tcaacgggtg tgccaatggc cactttccag gtggcaaagc    60

ccgttgagct tctcaaatct gagaagtggc actgagacgg gatccatccg tctccttttt    120

tccagatctc ctgtcttaaa aagttgccca cacggttacc ggtgaaaggt ccaccgtttc    180

gggcaactcg aagagtttag actcttcacc gtgactctgc cctaggtagg cagaggaaaa    240

aa                                                                    242

<210>	8
<211>	20
<212>	DNA
<213>	Artificial Sequence

<220>
<223>	Synthetic (hHPRT1-E2-1F primer)

<400>	8
gtgctgggat tacacgtgtg	20

<210>	9
<211>	20
<212>	DNA
<213>	Artificial Sequence

<220>
<223>	Synthetic (hHPRT1-E2-1R primer)

<400>	9
tgattcagcc ccagtccatt	20

<210>	10
<211>	57
<212>	DNA
<213>	Artificial Sequence

<220>
<223>	Synthetic (hHPRT1-E2-deepF primer)

<400>	10
acactctttc cctacacgac gctcttccga tcttgtttgt atcctgtaat gctctca	57

<210>	11
<211>	58
<212>	DNA
<213>	Artificial Sequence

<220>
<223>	Synthetic (hHPRT1-E2-deepR primer)

<400>	11
gtgactggag ttcagacgtg tgctcttccg atctcaaata gcaagtactc agaacagc	58

<210>	12

<211>	20
<212>	DNA
<213>	Artificial Sequence

<220>
<223>	Synthetic (hHPRT1-E3-1F primer)


<400>	12
agttcactcc agcctcaaca	20


<210>	13
<211>	20
<212>	DNA
<213>	Artificial Sequence

<220>
<223>	Synthetic (hHPRT1-E3-1R primer)


<400>	13
cccaagtccc aacagcaatt	20


<210>	14
<211>	58
<212>	DNA
<213>	Artificial Sequence

<220>
<223>	Synthetic (hHPRT1-E3-deepF)


<400>	14
acactctttc cctacacgac gctcttccga tctggaagtt taatgactaa gaggtgtt	58


<210>	15
<211>	60
<212>	DNA
<213>	Artificial Sequence

<220>
<223>	Synthetic (hHPRT1-E3-deepR primer)


<400>	15
gtgactggag ttcagacgtg tgctcttccg atctagctct tcagtctgat aaaatctaca	60

60


<210>	16
<211>	20

<212>	DNA
<213>	Artificial Sequence

<220>
<223>	Synthetic (hCCR5-1-1F primer)


<400>	16
gtttgcattc atggagggca	20


<210>	17
<211>	20
<212>	DNA
<213>	Artificial Sequence

<220>
<223>	Synthetic (hCCR5-1-1R primer)


<400>	17
gaccagcccc aagatgacta	20


<210>	18
<211>	56
<212>	DNA
<213>	Artificial Sequence

<220>
<223>	Synthetic (hCCR5-1-deepF primer)


<400>	18
acactctttc cctacacgac gctcttccga tctcatcctc atcctgataa actgca	56


<210>	19
<211>	54
<212>	DNA
<213>	Artificial Sequence

<220>
<223>	Synthetic (hCCR5-1-deepR primer)


<400>	19
gtgactggag ttcagacgtg tgctcttccg atctcaaaca cagcatggac gaca	54


<210>	20
<211>	20
<212>	DNA
<213>	Artificial Sequence

<220>
<223>    Synthetic (hCCR5-2-1F primer)

<400>    20
ccttctgggc tcactatgct                                                    20

<210>    21
<211>    20
<212>    DNA
<213>    Artificial Sequence

<220>
<223>    Synthetic (hCCR5-2-1R primer)

<400>    21
gcgtcatccc aagagtctct                                                    20

<210>    22
<211>    53
<212>    DNA
<213>    Artificial Sequence

<220>
<223>    Synthetic (hCCR5-2-deepF primer)

<400>    22
acactctttc cctacacgac gctcttccga tcttttgcgt ctctcccagg aat               53

<210>    23
<211>    56
<212>    DNA
<213>    Artificial Sequence

<220>
<223>    Synthetic (hCCR5-2-deepR primer)

<400>    23
gtgactggag ttcagacgtg tgctcttccg atctgatggt gaagataagc ctcaca            56

<210>    24
<211>    20
<212>    DNA
<213>    Artificial Sequence

<220>
<223>    Synthetic (hDNMT1-1-1F primer)

<400>    24
ttgggaatgt gggtactgct                                                    20

<210>    25
<211>    20
<212>    DNA
<213>    Artificial Sequence

<220>
<223>    Synthetic (hDNMT1-1-1R primer)

<400>    25
cagaggttac agtgagccga                                                    20

<210>    26
<211>    56
<212>    DNA
<213>    Artificial Sequence

<220>
<223>    Synthetic (hDNMT1-1-deepF primer)

<400>    26
acactctttc cctacacgac gctcttccga tcttgtgttt ccttgttctc tgacac            56

<210>    27
<211>    54
<212>    DNA
<213>    Artificial Sequence

<220>
<223>    Synthetic (hDNMT1-1-deepR

<400>    27
gtgactggag ttcagacgtg tgctcttccg atctgtttgg aaaggggttt gggg              54

<210>    28
<211>    20
<212>    DNA
<213>    Artificial Sequence

<220>
<223>    Synthetic (hDNMT1-2-1F primer)

<400>    28
taggcatgta ccaccacacc                                                    20

<210>	29
<211>	20
<212>	DNA
<213>	Artificial Sequence

<220>
<223>	Synthetic (hDNMT1-2-1R primer)

<400>	29
tcagtgccca ccgataatca	20

<210>	30
<211>	53
<212>	DNA
<213>	Artificial Sequence

<220>
<223>	Synthetic (hDNMT1-2-deepF primer)

<400>	30
acactctttc cctacacgac gctcttccga tcttgtcaag tgggtgcttc tct	53

<210>	31
<211>	55
<212>	DNA
<213>	Artificial Sequence

<220>
<223>	Synthetic (hDNMT1-2-deepR primer)

<400>	31
gtgactggag ttcagacgtg tgctcttccg atcttgtaga acttgttgac ccgga	55

<210>	32
<211>	24
<212>	DNA
<213>	Artificial Sequence

<220>
<223>	Synthetic (AacC2c1-HPRT1-TS1)

<400>	32
ttttgcatac ctaatcatta tgct	24

<210>	33

<211>	24
<212>	DNA
<213>	Artificial Sequence

<220>
<223>	Synthetic (AacC2c1-HPRT1-TS2)

<400>	33
attatgctga ggatttggaa aggg	24

<210>	34
<211>	24
<212>	DNA
<213>	Artificial Sequence

<220>
<223>	Synthetic (AacC2c1-HPRT1-TS3)

<400>	34
attaggtatg caaaataaat caag	24

<210>	35
<211>	24
<212>	DNA
<213>	Artificial Sequence

<220>
<223>	Synthetic (AacC2c1-HPRT1-TS4)

<400>	35
tttccaaatc ctcagcataa tgat	24

<210>	36
<211>	24
<212>	DNA
<213>	Artificial Sequence

<220>
<223>	Synthetic (AacC2c1-HPRT1-TS5)

<400>	36
attgtagccc tctgtgtgct caag	24

<210>	37
<211>	24
<212>	DNA
<213>	Artificial Sequence

<220>
<223>	Synthetic (AacC2c1-HPRT1-TS6)

<400>	37
tttgctgacc tgctggatta catc	24

<210>	38
<211>	24
<212>	DNA
<213>	Artificial Sequence

<220>
<223>	Synthetic (AacC2c1-HPRT1-TS7)

<400>	38
cttgagcaca cagagggcta caat	24

<210>	39
<211>	24
<212>	DNA
<213>	Artificial Sequence

<220>
<223>	Synthetic (AacC2c1-HPRT1-TS8)

<400>	39
tttgatgtaa tccagcaggt cagc	24

<210>	40
<211>	24
<212>	DNA
<213>	Artificial Sequence

<220>
<223>	Synthetic (AacC2c1-CCR5-TS1)

<400>	40
tttccttctt actgtcccct tctg	24

<210>	41
<211>	24
<212>	DNA
<213>	Artificial Sequence

<220>
<223>	Synthetic (AacC2c1-CCR5-TS2)

<400>    41
cttactgtcc ccttctgggc tcac                                              24

<210>    42
<211>    24
<212>    DNA
<213>    Artificial Sequence

<220>
<223>    Synthetic (AacC2c1-CCR5-TS3)

<400>    42
atttccaaag tcccactggg cggc                                              24

<210>    43
<211>    24
<212>    DNA
<213>    Artificial Sequence

<220>
<223>    Synthetic (AacC2c1-CCR5-TS4)

<400>    43
gttgacacat tgtatttcca aagt                                              24

<210>    44
<211>    24
<212>    DNA
<213>    Artificial Sequence

<220>
<223>    Synthetic (AacC2c1-CCR5-TS5)

<400>    44
tttccagaca ttaaagatag tcat                                              24

<210>    45
<211>    24
<212>    DNA
<213>    Artificial Sequence

<220>
<223>    Synthetic (AacC2c1-CCR5-TS6)

<400>    45

attaaagata gtcatcttgg ggct                                                24

<210>    46
<211>    24
<212>    DNA
<213>    Artificial Sequence

<220>
<223>    Synthetic (AacC2c1-CCR5-TS7)

<400>    46
cttgtcatgg tcatctgcta ctcg                                                24

<210>    47
<211>    24
<212>    DNA
<213>    Artificial Sequence

<220>
<223>    Synthetic (AacC2c1-CCR5-TS8)

<400>    47
gtttttagga ttcccgagta gcag                                                24

<210>    48
<211>    24
<212>    DNA
<213>    Artificial Sequence

<220>
<223>    Synthetic (AacC2c1-DNMT1-TS1)

<400>    48
tttgtccttg gagaacggtg ctca                                                24

<210>    49
<211>    24
<212>    DNA
<213>    Artificial Sequence

<220>
<223>    Synthetic (AacC2c1-DNMT1-TS2)

<400>    49
gttctccaag gacaaatctt tatt                                                24

<210>	50
<211>	24
<212>	DNA
<213>	Artificial Sequence

<220>
<223>	Synthetic (AacC2c1-DNMT1-TS3)


<400>	50
cttacaaccg ggaagtgaat ggac	24


<210>	51
<211>	24
<212>	DNA
<213>	Artificial Sequence

<220>
<223>	Synthetic (AacC2c1-DNMT1-TS4)


<400>	51
attcacttcc cggttgtaag catg	24


<210>	52
<211>	24
<212>	DNA
<213>	Artificial Sequence

<220>
<223>	Synthetic (AacC2c1-DNMT1-TS5)


<400>	52
tttccggtag tgctctgggt acag	24


<210>	53
<211>	24
<212>	DNA
<213>	Artificial Sequence

<220>
<223>	Synthetic (AacC2c1-DNMT1-TS6)


<400>	53
gttgctgcct ttgatgtagt cgga	24


<210>	54
<211>	24
<212>	DNA

<213>    Artificial Sequence

<220>
<223>    Synthetic (AacC2c1-DNMT1-TS7)


<400>    54
attggccgga tcaaagagat cttc                                              24


<210>    55
<211>    24
<212>    DNA
<213>    Artificial Sequence

<220>
<223>    Synthetic (AacC2c1-DNMT1-TS8)


<400>    55
cttctgtccc aagaagagca acgg                                              24

## Description

### Technical Field

The disclosure pertains to a genome-editing technique using C2c1 endonuclease and, specifically, to a genome-editing composition comprising C2c1 endonuclease and guide RNA and a genome-editing method using the same.

### Background Art

The CRISPR-Cas system is a protecting mechanism, found in bacteria and archaea, which confers resistance to genetic elements. The CRISPR-Cas system is characterized by recognizing and cleaving specific target sequences of DNA or RNA by using a guide RNA capable of complementarily binding to foreign DNA or RNA and a Cas effector protein serving as a nuclease. Thanks to the characteristics, the system has recently been used as a gene editing technique capable of editing genes of higher organisms and thus has a wide variety of applications in basic and applied scientific fields.

The most widely used CRIPR gene-editing technique is based on the type 2 CRISPR-Cas9 system found in Streptococcus pyogenes. The CRISPR-Cas9 system derived from S. pyogenes is generally effective in the sense of the ability to induce gene editing at desired target sites at high efficiency, but within the limitation that the presence of 5'-NGG PAM sequence is required for the target sequence thereof. As one of the strategies for overcoming the limitation, extensive studies have been conducted into the plausibility of utilizing novel CRISPR-Cas systems or other types of gene editing systems from different species.

### Detailed Description of the Invention

### Technical Problem

An aspect provides a genome-editing composition comprising a C2c1-CRISPR system, a gene encoding the same (DNA (cDNA, rDNA, etc.) or RNA (mRNA, etc.)), or an expression vector carrying the gene.

Another aspect provides a genome-editing method comprising a step of introducing the genome-editing composition into a cell or an organism.

Another aspect provides a genetically modified cell obtained using the genome-editing composition.

Another aspect provides a genetically modified organism obtained from the genetically modified cell.

Another aspect provides a method for production of a transgenic animal, the method comprising a step of transplanting into an oviduct of a mammalian foster mother a mammalian embryo having the genome-editing composition introduced thereinto.

### Technical Solution

The C2c1-CRISPR system is a type VB CRISPR-Cas system found recently. As opposed to the SpCas9 system most utilized conventionally, the C2c1-CRISPR system is characterized by containing C2c1 protein, instead of Cas9, as an effector protein serving as a nuclease, recognizing 5'-TTN PAM sequence instead of than 5'-TTN PAM sequence, and forming a sticky end rather than a blunt end upon DNA cleavage.

Proposed in this description is a technique employing a C2c1 endonuclease for gene editing in eukaryotic cells such as human cells.

An aspect provides a genome-editing composition comprising a C2c1-CRISPR system, a gene encoding the same (DNA (cDNA, rDNA, etc.) or RNA (mRNA, etc.)), or an expression vector carrying the gene.

The C2c1-CRISPR system may contain a C2c1 protein (e.g., AacC2c1 protein) and a guide RNA (e.g., single-guide RNA (sgRNA)) capable of hybridizing with (having a complementary nucleotide sequence to) a target site of a target gene.

Accordingly, the genome-editing composition may comprise (1) C2c1 endonuclease, a gene coding therefor, or an expression vector carrying the gene, and (2) a guide RNA, a DNA coding therefor, or an expression vector carrying the same.

Another aspect provides a genome-editing method comprising a step of introducing (administering or injecting) the genome-editing composition into a cell or an organism. The cell may be a cell (e.g., eukaryotic cell) isolated from a living organism and the organism may be an eukaryotic organism (e.g., mammal) exclusive or inclusive of a human being.

The cell may be an eukaryotic cell such as an eukaryotic animal, an eukaryotic plant cell, or the like, and the organism may be an eukaryotic organism such as an eukaryotic animal or an eukaryotic plant.

Another aspect provides a genetically modified cell obtained using the genome-editing composition. The cell may be an eukaryotic cell. The cell may be a cell from an eukaryotic animal. In an embodiment, the cell may be a cell from a mammal inclusive or exclusive of a human being.

Another aspect provides a method for production of a transgenic animal, the method comprising a step of transplanting into an oviduct of a mammalian foster mother a mammalian embryo having the genome-editing composition introduced thereinto. The mammalian animal (foster mother) having the embryo transplanted into the oviduct thereof may be the same species as in the mammalian animal from which the embryo is derived.

Another aspect provides a genetically modified organism obtained from the genetically modified cell. The genetically modified organism may be generated by a method for generating a transgenic animal. The animal may be an eukaryotic animal, such as a mammal inclusive or exclusive of a human being.

The C2c1-CRISPR endonuclease is found in various bacterial species. A representative example is *Alicyclobacillus acidoterrestris-derived* C2c1-CRISPR endonuclease (hereinafter referred to as "AacC2c1"). Wild-type (WT) AacC2c1 is composed of 1129 amino acids (see SEQ ID NO: 1) and may be a protein encoded by the nucleotide sequence provided in the website https://www.addgene.org/browse/sequence/125305/ (a region from position 1771 to position 5160).

As used herein, the term "guide RNA" refers to an RNA that includes a targeting sequence hybridizable with a specific base sequence (target sequence) of a target site in a target gene and functions to associate with a nuclease, such as Cac1 protein, etc., and guide the nuclease to a target gene (or target site) in vitro or in vivo (or cells).

The guide RNA may be suitably selected depending on kinds of the nuclease to be complexed therewith and/or origin microorganisms thereof.

For example, the guide RNA may be at least one selected from the group consisting of:
CRISPR RNA (crRNA) including a region (targeting sequence) hybridizable with a target sequence;
trans-activating crRNA (tracrRNA) including a region interacting with a nuclease such as C2c1 protein, etc.; and
single guide RNA (sgRNA) in which main regions of crRNA and tracrRNA (e.g., a crRNA region including a targeting sequence or a tracrRNA region interacting with nuclease) are fused to each other.

In detail, the guide RNA may be a dual RNA including CRISPR RNA (crRNA) and trans-activating crRNA (tracrRNA) or a single guide RNA (sgRNA) including main regions of crRNA and tracrRNA.

The sgRNA may include a region (named "spacer region", "target DNA recognition sequence", "base pairing region", etc.) having a complementary sequence (targeting sequence) to a target sequence in a target gene (target site), and a hairpin structure for binding to a C2c1 protein. So long as it includes main regions of crRNA and tracrRNA and a complementary sequence to a target DNA, any guide RNA can be used in the present disclosure.

For editing a target gene, for example, the C2c1 protein requires two guide RNAs, that is, a CRISPR RNA (crRNA) having a nucleotide sequence hybridizable with a target site in the target gene and a trans-activating crRNA (tracrRNA) interacting with the C2c1 protein. In this context, the crRNA and the tracrRNA may be coupled to each other to form a crRNA:tracrRNA duplex or connected to each other via a linker so that the RNAs can be used in the form of a single guide RNA (sgRNA) in which a partial double strand is formed to make a folding structure suitable for binding the C2c1 protein. In one embodiment, when AacC2c1 is used, the sgRNA may form a structure in which the entirety or a part of the crRNA having a hybridizable nucleotide sequence is connected to the entirety or a part of the tracrRNA including an interacting region with the C2c1 protein.

The guide RNA, specially, crRNA or sgRNA, includes a targeting sequence complementary to a target sequence in a target gene and may contain one or more, for example, 1-10, 1-5, or 1-3 additional nucleotides at an upstream region of crRNA or sgRNA, particularly at the 5' end of sgRNA or the 5' end of crRNA in dual RNA. The additional nucleotide(s) may be guanine(s) (G), but are not limited thereto.

In one embodiment, the sgRNA for AacC2c1 may be represented by the following General Sequence Formula 1:
5'-**GUCUAGAGGACAGAAUUUUUCAACGGGUGUGCCAAUGGCCACUUUCCA GGUGGCAAAGCCCGUUGAGCUUCUCAAAU***CUGAGAAGUGGX₁X₂X₃*[(N)n]-3' (5'-[SEQ ID NO: 2]-[(N)n]-3'; General Sequence Formula 1)
wherein,
the region in bold is derived from tracrRNA,
the region in italics is derived from crRNA,
X₁, X₂, and X₃ are each a nucleotide independently selected from A, U, G, and C,
(N)n is a targeting sequence hybridizable with (having a complementary sequence to) a target sequence and consisting of n nucleotides that may be the same or different and are each independently selected from A, U, G, and C, wherein n is an integer of 17-23, 18-22, or 19-21 (e.g., 20).

In an embodiment, the guide RNA is a mutant resulting from the deletion of 1-15, 5-15, 10-15, or 12-15 nucleotides from the sequence of General Sequence Formula 1. In this context, the deleted nucleotide(s) may be 1-15, 5-15, 10-15, or 12-15 (e.g., 12 or 15) nucleotide(s) selected from the nucleotides of the underlined sequence (AGCUUCUCAAA) in General Sequence Formula 1.

In an embodiment, the sigRNA for AacC2c1 may be represented by General Sequence Formula 2, below, but is not limited thereto:
5'-GUCUAGAGGACAGAAUUUUUCAACGGGUGUGCCAAUGGCCACUUUCCAGG UGGCAAAGCCCGUUG**AGCUUCUCAAAUCUG***AGAAGUGGCAC*[(N)n]-3' (5'-[SEQ ID NO: 3]-[(N)n]-3'; General Sequence Formula 2).

In General Sequence Formula 2, n may be 20.

The crRNA or sgRNA may further contain 1 to 3 guanines (G) at the 5' end thereof (that is, the 5' end of the targeting sequence of crRNA).

The target sequence for the guide RNA may be about 17 to about 23, about 18 to about 22, or about 19 to about 21, for example, 20 consecutive nucleotides adjacent to the 5' end of PAM (Protospacer Adjacent Motif (5'-TTN-3' (N is A, T, G, or C)) on a target DNA.

As used herein, the term "the targeting sequence" of guide RNA, which is hybridizable with the target sequence for the guide RNA, refers to a nucleotide sequence having a sequence complementarity of 50 % or higher, 60 % or higher, 70 % or higher, 80 % or higher, 90 % or higher, 95 % or higher, 99 % or higher, or 100 % to a nucleotide sequence of a complementary strand to a DNA strand on which the target sequence exists (i.e., a PAM sequence (5'-TTN-3' (N is A, T, G, or C))). In an embodiment, the targeting sequence of guide RNA may be a sequence complementary to and hybridizable with a nucleotide sequence on the complementary strand to the target strand on which the PAM sequence exists.

In the description, a nucleic acid sequence (target sequence) at a target site is represented by that of the strand on which a PAM sequence exists between two DNA strands in a region of a target gene. In this regard, the DNA strand to which the guide RNA couples is complementary to a strand on which a PAM sequence exists. Hence, the targeting sequence included in the guide RNA has the same nucleic acid sequence as a sequence at an on-target site, with the exception that U is employed instead of T due to the RNA property. In other words, a targeting sequence of the guide RNA and a target sequence are represented by the same nucleic acid sequence with the exception that T and U are interchanged, in the description.

The C2c1 endonuclease and the guide RNA may be in the form of:
a ribonucleic acid protein in which the C2c1 endonuclease protein and the guide RNA are complexed with each other in vitro (assembled in advance in vitro) (in this regard, injected in the form of a complex into a body or cells and transported across cell membranes and/or nuclear membranes);
a mixture of the C2c1 endonuclease or an mRNA coding therefor, and the guide RNA; or
a plasmid carrying together DNAs coding respectively for the C2c1 endonuclease protein and the guide RNA or separate plasmids respectively carrying the DNAs.

The C2c1 protein may be linked to a cell penetrating peptide and/or a protein transduction domain. The protein transduction domain may be poly-arginine or an HIV-derived TAT protein, but is not limited thereto. Because there are various kinds of the cell penetrating peptide or the protein transduction domain in addition to the stated examples, a person skilled in the art may make application of various kinds without limitations to the examples.

In addition, the C2c1 protein or a gene coding therefor may further comprise a nuclear localization signal (NLS) sequence or a nucleic acid sequence coding therefor. Therefore, an expression cassette including a C2c1 protein-encoding gene may further comprise a regulatory sequence such as a promoter sequence for expressing the C2c1 protein and/or nuclease and optionally an NLS sequence. The NLS sequence is well known in the art and may have the amino acid sequence of PKKKRKV, but is not limited thereto. Particularly when the C2c1 protein or a ribonucleic acid protein containing the same is applied to eukaryotic cells and/or eukaryotic organisma, the NLS sequence may be needed.

The C2c1 protein or the nucleic acid coding therefor may be linked to a tag for isolation and/or purification or a nucleic acid coding for the tag. For example, the tag may be selected from the group consisting of small peptide tags, such as His tag, Flag tag, S tag, etc., GST (Glutathione S-transferase) tag, and MBP (Maltose binding protein) tag, but is not limited thereto.

As used herein, the term "genome editing" means deletion and/or insertion of at least one nucleotide and/or substitution of a different nucleotide for an original nucleotide in a target gene in a genome, and/or the inactivation (dysfunction or functional loss) of the target gene.

The C2c1 protein breaks a DNA double strand at a vicinity of the PAM sequence, forming a sticky end, which results in different lengths for the two strands at the ends, because of different cutting positions on the two strands. AcsC2c1 may break a DNA double strand at a position 5-25 nt (nucleotides), 5-20 nt, 10-25 nt, 10-20 nt, or 15-20 nt distant from the PAM sequence in the 3' direction on the PAM sequence-bearing strand (5' or 3' end of the nucleotide at the position; e.g., between nucleotides at positions 17 and 18 from the PAM in the 3' direction) and at a position 15-30 nt (nucleotides), 15-25 nt, 20-30 nt, or 20-25 nt distant from a sequence complementary to the PAM sequence in the 5' direction on the other stand (complementary to the PAM sequence-bearing strand), forming a sticky end. The break positions may vary depending on the positions of target sequences.

The cells may be prokaryotic or eukaryotic (eukaryotic animal cells or eukaryotic plant cells) and particularly may be cells from eukaryotic animals such as mammals inclusive of humans. The cells may exist in a living body or may be cells isolated from a living body.

The introduction of genes into cells may be conducted by introducing an expression vector carrying the C2c1-encoding gene and the guide RNA-encoding gene together or expression vectors respectively carrying the genes into cells in a conventional process (electroporation, lipofection, etc.) and expressing the genes therein, or by directly introducing a C2c1-guide RNA complex assembled in vitro in advance or a mixture of a C2c1 protein or mRNA coding therefor and a guide RNA into cells through electroporation, lipofection, microinjection, etc. without use of an expression vector. The introduction may be conducted in vitro for cells isolated from a living body.

As used herein,
"target gene" means a gene as an object to which base editing (or base mutation) is applied, and
"target site" or "target region" means a site or region at which a target-specific nuclease performs base editing in a target gene. In one embodiment, when the target-specific nuclease includes an RNA-guided nuclease (RNA-guided engineered nuclease; RGEN), the target site or target region is intended to be a gene site (a double strand, or any one single strand in a double strand) which is located adjacent to the 5'- and/or 3'-end of the RNA-guided nuclease-recognized sequence (PAM sequence) in a target gene and has a maximum length of about 50 bp or about 40 bp.

In one embodiment, when the target-specific nuclease includes an RNA-guided nuclease, a guide RNA containing a targeting sequence may be included together with the RNA-guided nuclease. The "targeting sequence" may be a guide RNA site including a base sequence complementary to (hybridizable with) a consecutive base sequence of about 15 to about 30 nucleotides (nt), about 15 to about 35 nt, about 17 to about 23 nt, or about 18 to about 22 nt, e.g., about 20 nt on a target site. The base sequence on a target site, complementary to the targeting sequence, is called a "target sequence". The "target sequence" may mean a consecutive base sequence of about 15 nt to about 30 nt, about 15 nt to about 25 nt, about 17 nt to about 23 nt, or about 18 nt to about 22 nt, for example, about 20 nt located adjacent to the 5'- and/or 3'-end of a PAM sequence recognized by an RNA-guided nuclease.

### Advantageous Effects

In the description, it is first suggested that the type VB CRISPR-C2c1 system can be utilized in human genome editing. The C2c1-CRISPR derived from *Alicyclobacillus acidoterrestris* was representatively employed. Even though the bacterium is a thermophile optimized at high temperatures, the C2c1-CRISPR derived therefrom was found to operate at high efficiency for specific target sequences of human HPRT1 gene even in a 37°C condition. Given, a C2c1-CRISPR system derived from mesophilic bacteria that live in a temperature condition suitable for human genome editing other than from thermophiles is expected to guarantee a novel gene editing tool capable of genome editing at higher efficiency, making contribution to expanding a genome editing platform using CRISPR systems.

### Brief Description of the Drawings

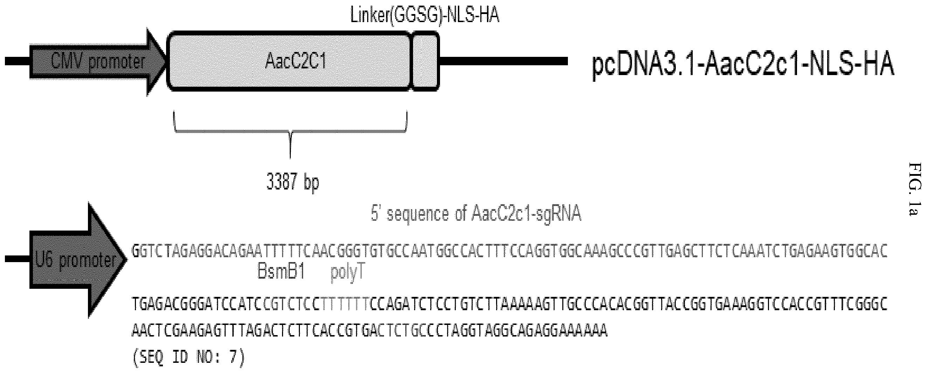

FIGS. 1a to 1c show configurations of AacC2c1-CRISPR system expression vectors and results of the protein expression using the same, presenting schematic diagrams of 1a an AacC2c1 protein expression vector and an Aac-sgRNA cloning vector (1a) and AacC2c1 protein expression results (1b) (left panel: 1^{st} antibody, HA tag; and right panel: 1^{st} antibody, GAPDH).

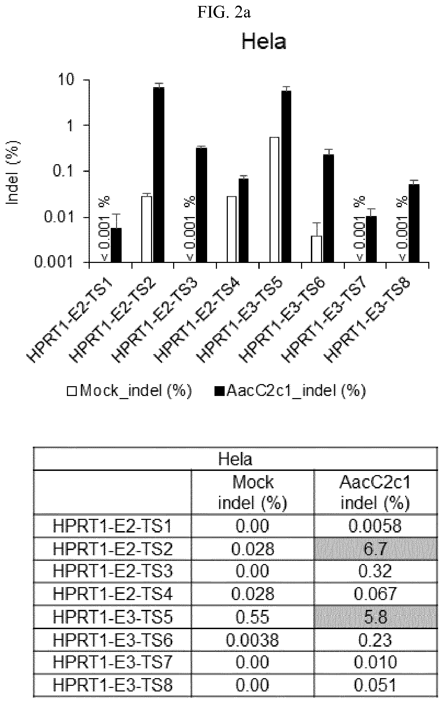

FIGS. 2a to 2d show human HPRT1 gene editing results obtained by using the AacC2c1 and sgRNA expression vectors, presenting graphs of human HPRT1 target gene editing results (Indel(%)) obtained using AacC2c1 for Hela cells (2a) and HEK293T cells (2b) (Biological replicate (N=3)), 1^{st} NGS analysis results for HPRT1-TS2 and HPRT1-TS5 (2c) (PAM sequence in blue; AacC2c1 target sequences in upper cases; sequences around the AacC2c1 target in lower cases; and WT, wild type), and photographs of colony formation assay results obtained through crystal violet staining (2d) (after transfection of AacC2c1 and sgRNA expression vectors thereinto, Hela cells were seeded and subjected to 6-thioguanine (6-TG) selection. Two weeks later, colonies of the cells in which a HPRT1 gene was knocked out by mutation were identified by crystal violet staining).

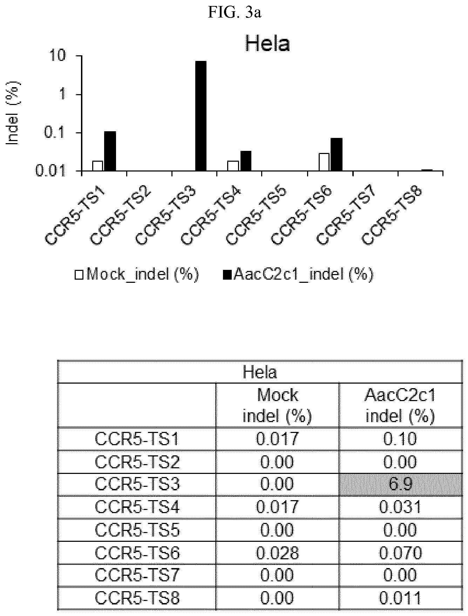

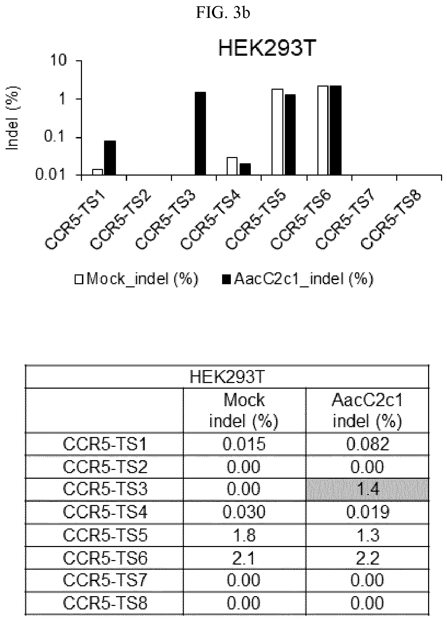

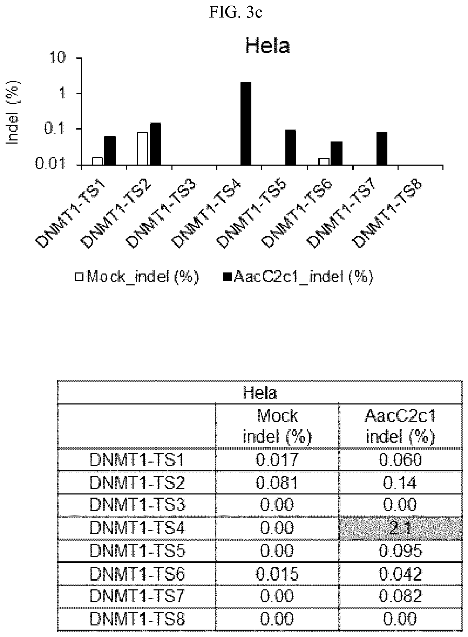

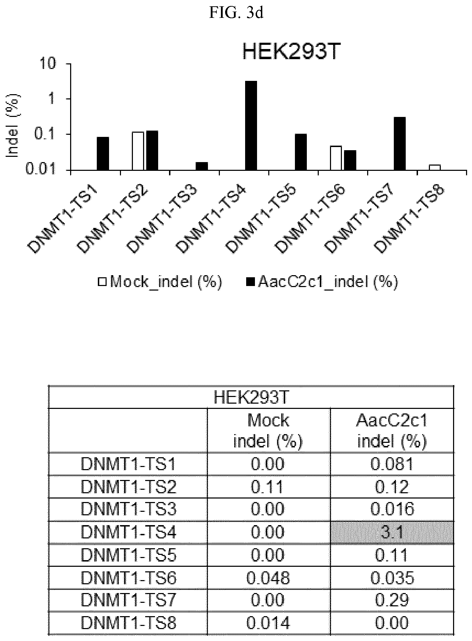

FIGS. 3a to 3e show gene editing results obtained by applying AacC2c1 and sgRNA expression vectors to human CCR5 and DNMT1 genes, presenting graphs of human CCR5 target gene editing results (Indel(%)) obtained using AacC2c1 for Hela cells (3a) and HEK293T cells (3b) (Biological replicate (N=1)), graphs of human DNMT1 target gene editing results (Indel(%)) obtained using AacC2c1 or Hela cells (3c) and HEK293T cells (3d) (Biological replicate (N=1)), and 1^{st} NGS analysis results for CCR5-TS3 and DNMT1-TS4 (3e) (PAM sequence in blue; AacC2c1 target sequences in upper cases; sequences around the AacC2c1 target in lower cases; and WT, wild type).

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLE 1: Vector Construction

With pRSFDuet1-His6-Sumo-AacC2C1-CN plasmid (pRSF-Duet-1-His6-SUMO-AacC2c1 plasmid, granted by Pf. Dinshaw Patel group, was added with an NLS(PKKKRKV)-HA tag sequence at the C-terminal of AacC2c1) serving as a template, the coding sequence of AacC2c1 was amplified by PCR (CMV-AacC2c1-CN-1F-Hind3:
GCTGGCTAGCGTTTAAACTTAAGCTTGGTACCGCCACCATGGCAGTGAAAAG CATCAAAG (SEQ ID NO: 5), CMV-AacC2c1-CN-1R-BamH1: AACATCGTATGGGTAGGATCCTCAGGCGTAGTCGGGCAC (SEQ ID NO: 6), phusion DNA polymerase was used), and the PCR product was inserted into pcDNA3.1 backbone vector (Invitrogen, V790-20) by Gibson cloning.

In order to be optimized for human cell expression and genome editing, the above-prepared AacC2c1 coding sequence was used, together with the CMV promoter and the nuclear localization signal (PKKKRKV), to construct an expression cassette. An AacC2c1 expression vector was constructed under the design that the AacC2c1 gene is transcribed under the CMV promoter suitable for human cell expression and the SV40 nucleus localization signal (PKKKRKV) and the HA tag are positioned at the C terminal of the AacC2c1 coding sequence (pcDNA3.1-AacC2c1-NLS-HA; FIG. 1a).

An Aac-sgRNA cloning vector was constructed in which the sgRNA binding complementarily to a target sequence was transcribed under the human U6 promoter. For Aac-sgRNA, the sequence in the 5'-terminal sequence was used in common while 20 consecutive nucleotides at the 3'-terminal site of sgRNA were substituted in accordance with target sequences (see Table 2) to construct sgRNA that acted at specific target positions (see General Sequence Formula 2). The Aac-sgRNA cloning vector was constructed on the basis of a U6-Sp-sgRNA cloning vector (see FIG. 1a).

### EXAMPLE 2: Cell Test

Hela cells (ATCC) and HEK293T cells (ATCC) were both tested in 24-well-plate scales. On the day before transfection, cells were seeded. The next day, transfection was performed at 70-80% confluency. Using lipofectamine2000 (Invitrogen), 500 ng of each of the AacC2c1 and Aac-sgRNA vectors prepared in Example 1 was delivered into the cells. 72 hours after transfection, genomic DNA samples were extracted using DNeasy Blood & Tissue kit (Qiagen) and subjected to the following additional analyses.

### EXAMPLE 3: Targeted deep sequencing

A Deep sequencing library was prepared by PCR. TruSeq HT Dual Index primers were used to assign respective I.D.s to samples, followed by analysis with the aid of MiniSeq 300 cycles kit (Illumina). Primer sequences used in the deep sequencing are summarized in Table 1, below:

**[Table 1]**

| Locus | Primer name | SE Q ID NO | Primer sequence | Target site |
|---|---|---|---|---|
| HPRT1 | hHPRT1-E2-1F | 8 | gtgctgggattacacgtgtg | HPRT1-E2-TS1, HPRT1-E2-TS2, HPRT1-E2-TS3, HPRT1-E2-TS4 |
| | hHPRT1-E2-1R | 9 | tgattcagccccagtccatt | |
| | hHPRT1-E2-deepF | 10 | ACACTCTTTCCCTACACGACGCTCTT CCGATCTtgtttgtatcctgtaatgctctca | |
| | hHPRT1-E2-deepR | 11 | GTGACTGGAGTTCAGACGTGTGCTC TTCCGATCTcaaatagcaagtactcagaacagc | |
| | hHPRT1-E3-1F | 12 | agttcactccagcctcaaca | HPRT1-E2-TS5, HPRT1-E2-TS6, HPRT1-E2-TS7, HPRT1-E2-TS8 |
| | hHPRT1-E3-1R | 13 | cccaagtcccaacagcaatt | |
| | hHPRT1-E3-deepF | 14 | ACACTCTTTCCCTACACGACGCTCTT CCGATCTggaagtttaatgactaagaggtgtt | |
| | hHPRT1-E3-deepR | 15 | GTGACTGGAGTTCAGACGTGTGCTC TTCCGATCTagctcttcagtctgataaaatctaca | |
| CCR5 | hCCR5-1-1F | 16 | gtttgcattcatggagggca | CCR5-TS1, CCR5-TS2, CCR5-TS3, CCR5-TS4 |
| | hCCR5-1-1R | 17 | gaccagccccaagatgacta | |
| | hCCR5-1-deepF | 18 | ACACTCTTTCCCTACACGACGCTCTT CCGATCTcatcctcatcctgataaactgca | |
| | hCCR5-1-deepR | 19 | GTGACTGGAGTTCAGACGTGTGCTC TTCCGATCTcaaacacagcatggacgaca | |
| | hCCR5-2-1F | 20 | ccttctgggctcactatgct | CCR5-TS5, CCR5-TS6, CCR5-TS7, CCR5-TS8 |
| | hCCR5-2-1R | 21 | gcgtcatcccaagagtctct | |
| | hCCR5-2-deepF | 22 | ACACTCTTTCCCTACACGACGCTCTT CCGATCTtttgcgtctctcccaggaat | |
| | hCCR5-2-deepR | 23 | ACACTCTTTCCCTACACGACGCTCTT CCGATCTtttgcgtctctcccaggaat | |
| DNMT1 | hDNMT1-1-1F | 24 | ttgggaatgtgggtactgct | DNMT1-TS1, DNMT1-TS2, DNMT1-TS3, DNMT1-TS4 |
| | hDNMT1-1-1R | 25 | cagaggttacagtgagccga | |
| | hDNMT1-1-deepF | 26 | ACACTCTTTCCCTACACGACGCTCTT CCGATCTtgtgtttccttgttctctgacac | |
| | hDNMT1-1-deepR | 27 | GTGACTGGAGTTCAGACGTGTGCTC TTCCGATCTgtttggaaaggggtttgggg | |
| | hDNMT1-2-1F | 28 | taggcatgtaccaccacacc | DNMT1-TS5, DNMT1-TS6, DNMT1-TS7, DNMT1-TS8 |
| | hDNMT1-2-1R | 29 | tcagtgcccaccgataatca | |
| | hDNMT1-2-deepF | 30 | ACACTCTTTCCCTACACGACGCTCTT CCGATCTtgtcaagtgggtgcttctct | |
| | hDNMT1-2-deepR | 31 | GTGACTGGAGTTCAGACGTGTGCTC TTCCGATCTtgtagaacttgttgacccgga | |

### EXAMPLE 4: 6TG Selection

After transfection of the AacC2c1 vector and the sgRNA vector thereinto in 24-well-plate scales, the cells were passaged to secure a sufficient number. One week after transfection, 1.5X₁06 cells were seeded in a 100 pi dish. From the next day, selection was started in a 6 µM 6TG (6-thioguanine) condition. Two weeks later, colonies of the cells in which the HPRT1 gene had been knocked out (KO) by mutation were identified by crystal violet staining.

### EXAMPLE 5: Confirmation of AacC2c1 Expression

Prior to genome editing, examination was made to see whether the AacC2c1 protein was expressed in human cells. In this regard, the pcDNA3.1-AacC2c1-NLS-HA plasmid was transfected into HEK293T cells with the aid of lipofectamine2000 (see Example 4). Twenty four hours after transfection, a whole cell lysate (WCL) was taken and assayed for AacC2c1 expression by a Western blotting method using a 1^{st} antibody specifically recognizing an HA tag present at the C-terminus of the AacC2c1 protein so as to specifically observe only the AacC2c1 protein.

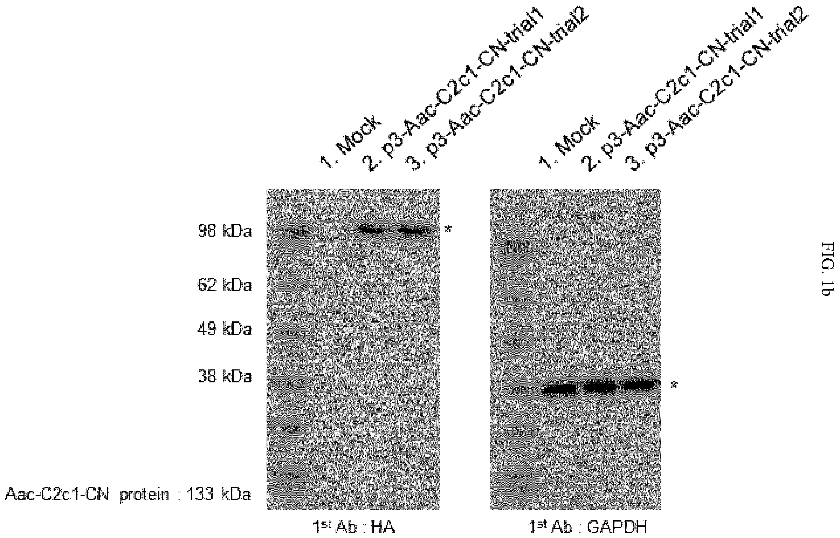

The results are depicted in FIG. 1b (left panel: 1^{st} antibody, HA tag; right panel: 1^{st} antibody, GAPDH). As shown in FIG. 1b, when the 1^{st} antibody specifically recognizing an HA tag was used, a protein was detected at 133 kDa in the p3-Aac-C2c1-CN plasmid-transfected experiment group, but not in the mock control.

### EXAMPLE 6: Genome Editing Using AacC2c1

Because the AacC2c1 protein was identified to be expressed in human cells (HEK293T cells) in Example 5, an examination was made to test whether human genome editing could be conducted with AacC2c1. A human HPRT1 gene was selected as a target gene. Experimental preference was given to HPRT1 gene because when complete KO was introduced by gene editing, a KO clone could be identified on a phenotype basis through 6-thioguanine (6-TG) selection and crystal violet staining.

AacC2c1 target sequences were selected for human HPRT1 gene and exon sequences of CCR5 and DNMT1 genes (Table 2), and respective Aac-sgRNA vectors corresponding to the target sequences were constructed (see Example 1).

**[Table 2]**

| | TARGET SITE (5'-**nTTN**(nPAM)-X₂0) | SEQ ID NO |
|---|---|---|
| AacC2c1-HPRT1-TS1 | **tTTT**GCATACCTAATCATTATGCT | 32 |
| AacC2c1-HPRT1-TS2 | **aTTA**TGCTGAGGATTTGGAAAGGG | 33 |
| AacC2c1-HPRT1-TS3 | **aTTA**GGTATGCAAAATAAATCAAG | 34 |
| AacC2c1-HPRT1-TS4 | **tTTC**CAAATCCTCAGCATAATGAT | 35 |
| AacC2c1-HPRT1-TS5 | **aTTG**TAGCCCTCTGTGTGCTCAAG | 36 |
| AacC2c1-HPRT1-TS6 | **tTTG**CTGACCTGCTGGATTACATC | 37 |
| AacC2c1-HPRT1-TS7 | **cTTG**AGCACACAGAGGGCTACAAT | 38 |
| AacC2c1-HPRT1-TS8 | **tTTG**ATGTAATCCAGCAGGTCAGC | 39 |
| AacC2c1-CCR5-TS1 | **tTTC**CTTCTTACTGTCCCCTTCTG | 40 |
| AacC2c1-CCR5-TS2 | **cTTA**CTGTCCCCTTCTGGGCTCAC | 41 |
| AacC2c1-CCR5-TS3 | **aTTT**CCAAAGTCCCACTGGGCGGC | 42 |
| AacC2c1-CCR5-TS4 | **gTTG**ACACATTGTATTTCCAAAGT | 43 |
| AacC2c1-CCR5-TS5 | **tTTC**CAGACATTAAAGATAGTCAT | 44 |
| AacC2c1-CCR5-TS6 | **aTTA**AAGATAGTCATCTTGGGGCT | 45 |
| AacC2c1-CCR5-TS7 | **cTTG**TCATGGTCATCTGCTACTCG | 46 |
| AacC2c1-CCR5-TS8 | **gTTT**TTAGGATTCCCGAGTAGCAG | 47 |
| AacC2c1-DNMT1-TS1 | **tTTG**TCCTTGGAGAACGGTGCTCA | 48 |
| AacC2c1-DNMT1-TS2 | **gTTC**TCCAAGGACAAATCTTTATT | 49 |
| AacC2c1-DNMT1-TS3 | **cTTA**CAACCGGGAAGTGAATGGAC | 50 |
| AacC2c1-DNMT1-TS4 | **aTTC**ACTTCCCGGTTGTAAGCATG | 51 |
| AacC2c1-DNMT1-TS5 | **tTTC**CGGTAGTGCTCTGGGTACAG | 52 |
| AacC2c1-DNMT1-TS6 | **gTTG**CTGCCTTTGATGTAGTCGGA | 53 |
| AacC2c1-DNMT1-TS7 | **aTTG**GCCGGATCAAAGAGATCTTC | 54 |
| AacC2c1-DNMT1-TS8 | **cTTC**TGTCCCAAGAAGAGCAACGG | 55 |

| | | |
|---|---|---|
| (X₂0 regions corresponding to the sequences except n(PAM) account for target sequences) AacC2c1 and Aac-sgRNA expression vectors were delivered into Hela cells and HEK293T cells with the aid of lipofectamine2000. 72 Hours after vector delivery, genomic DNA was isolated, and analyzed by targeted deep sequencing for gene editing efficiency on on-target sequences (introduced indel frequency (%)). For assessing the statistical significance of the introduced indels, targeted deep sequencing was performed on mock controls, which had not been treated with the AacC2c1 and Aac-sgRNA expression vectors. The experiment procedures were repeated three times in total. | | |

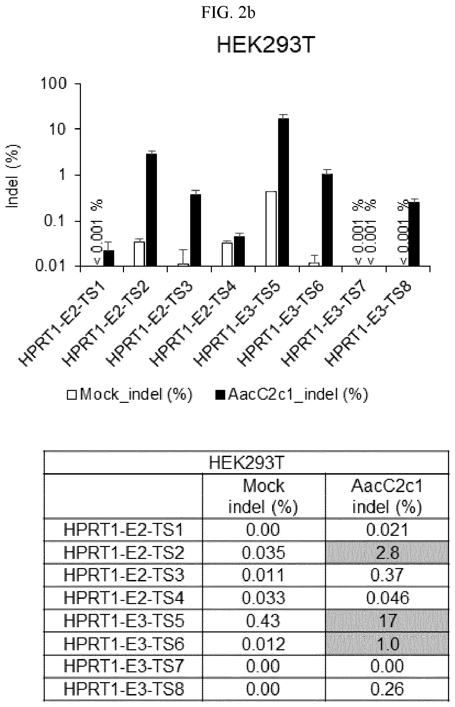

Analysis results are depicted in FIGS. 2a (Hela cell) and 2b (HEK293T cell). As shown in FIGS. 2a and 2b, HPRT1-TS2 and HPRT1-TS5 were respectively observed to introduce indel at frequencies of as high as 6.7 % and 5.8 % on average into the on-target sequences of Hela cells and at frequencies of as high as 2.8 % and 17 % on average into the target-sequences of HEK293T cells.

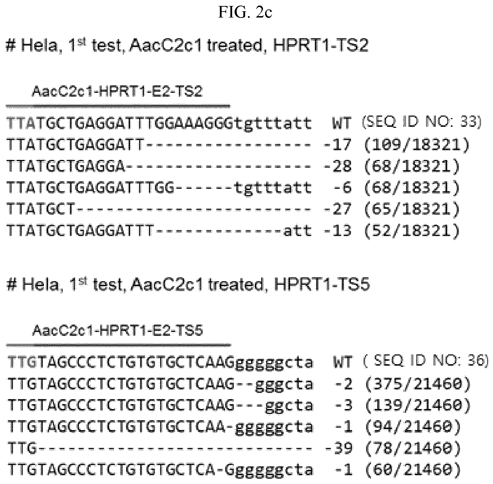

In addition, targeted deep sequencing raw data were analyzed to examine how indels were mainly introduced into on-target sequences. The results are depicted in FIG. 2c. As can be seen in FIG. 2c, the mutation pattern is deletion that was made mainly at 3' terminal regions of the on-target sequences (FIG. 2c).

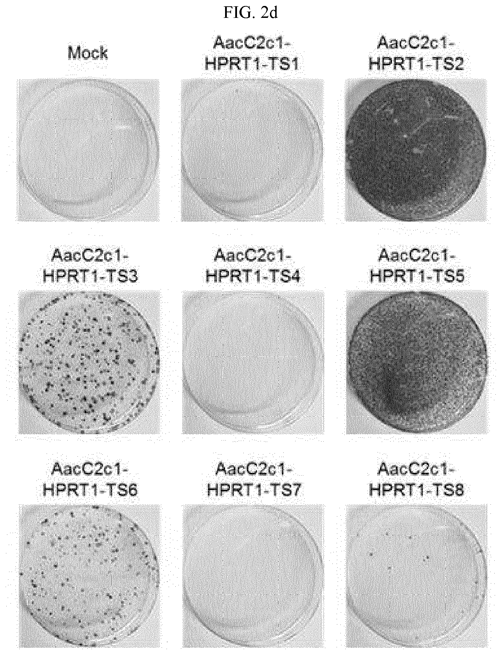

In order to more clearly prove the results obtained by the genetic analysis, the Hela cells to which the AacC2c1 and Aac-sgRNA expression vectors were delivered were subjected to 6-TG selection and crystal violet staining to investigate HPRT1 gene-knockout cells on a phenotype basis. In greater detail, the AacC2c1 and sgRNA expression vectors were transfected into Hela cells which were then seeded and subjected to 6-thioguanine (6-TG) selection. Two weeks later, colonies of the cells in which HPRT1 gene was knocked out by mutation were detected by crystal violet staining. The results are depicted in FIG. 2d. As shown in FIG. 2d, the cells to which the Aac-sgRNA and AacC2c1 plasmids corresponding respectively to HPRT1-TS2 and HPRT1-TS5 were delivered had the HPRT1 gene completely knocked out therein, and thus formed a lot of colonies as detected by 6-TG selection and appeared intensively stained upon crystal violet staining.

Examination was made to investigate whether the AacC2c1-CRISPR-Cas endonucleases could make gene editing on target sequences of genes other than HPRT1 gene. To this end, CCR5 and DNMT1 genes were additionally selected. For each of the target genes, 8 AacC2c1 target sequences were selected (Table 2). Aac-sgRNA vectors corresponding respectively to the target sequence were delivered, together with the AacC2c1 expression plasmid, into Hela cells and HEK293T cells. 72 hours after delivery, genomic DNA was extracted and analyzed for the introduction of indels into on-target sequences to measure indel frequencies.

The indel frequencies thus obtained are depicted in FIGS. 3a (indel frequencies on CCR5 target regions in Hela cells), 3b (indel frequencies on CCR5 target regions in HEK293T cells), 3c (indel frequencies on DNMT1 target regions in Hela cells), and 3d (indel frequencies on DNMT1 target regions in HEK293T cells). As can be seen in FIGS. 3a to 3d, indels were introduced into the CCR5-TS3 target region at frequencies of 6.9 % for Hela and 1.4 % for HEK293T cells and into the DNMT1-TS4 region at frequencies of 2.1 % for Hela cells and 3.1 % for HEK293T cells.

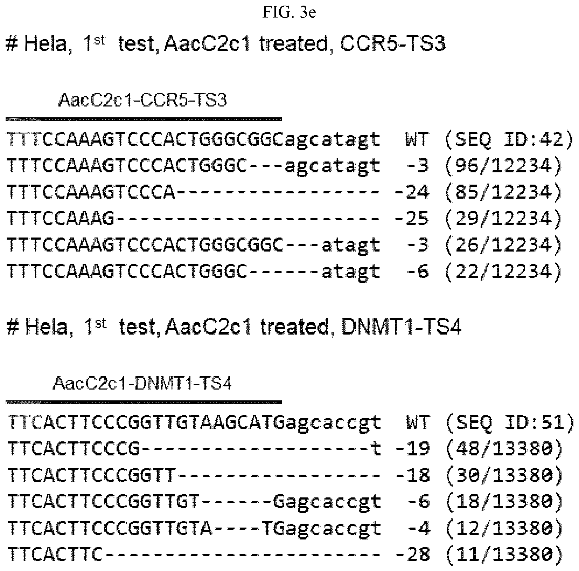

In addition, 1^{st} NGS (targeted deep sequencing) was performed for CCR5-TS3 and DNMT1-TS4 and the result is depicted in FIG. 3e. As shown in FIG. 3e, both CCR5-TS3 and DNMT1-TS4 were observed to have indels introduced into the target sequences therein.

Based on phenotype analysis results, genetic analysis confirmed that the wild-type AacC2c1-CRISPR system can be used for human genome editing and operates for specific target sequence locations at high yield.

## Claims

1. A genome editing composition, comprising:
a C2c1 endonuclease, a gene coding therefor, or an expression vector carrying the gene; and
a guide RNA, a DNA coding therefor, or an expression vector carrying the DNA.

2. The genome editing composition of claim 1, wherein the C2c1 endonuclease is derived from *Alicyclobacillus acidoterrestris.*

3. The genome editing composition of claim 2, wherein the guide RNA is represented by the following General Sequence Formula 1 or is a mutant resulting from the deletion of 1-15 nucleotides from the sequence of General Sequence Formula 1:
5'GUCUAGAGGACAGAAUUUUUCAACGGGUGUGCCAAUGGCCACUUUCCAGGUGGCAAAGCCCGUUGAGCUUCUCAAAUCUGAGAAGUGGX₁X₂X₃[(N)n]-3' (General Sequence Formula 1)
wherein,
X₁, X₂, and X₃ are each a nucleotide independently selected from A, U, G, and C, and
(N)n stands for n nucleotides that may be the same or different and are each independently selected from A, U, G, and C, wherein n is an integer of 17-23.

4. The genome editing composition of claim 3, wherein the deletion is performed on 1-15 nucleotides selected from the sequence AGCUUCUCAAA in General Sequence Formula 1.

5. The genome editing composition of claim 3, wherein the guide RNA is represented by the following General Sequence Formula 2:
5'GUCUAGAGGACAGAAUUUUUCAACGGGUGUGCCAAUGGCCACUUUCCAGGUGGCAAAGCCCGUUGAGCUUCUCAAAUCUGAGAAGUGGCAC[(N)n]-3' (General Sequence Formula 2)
wherein,
(N)n represents n nucleotides which may be the same or different and are selected from A, U, G, and C wherein n is an integer of 17-23.

6. The genome editing composition of claim 5, wherein n is 20.

7. The genome editing composition of claim 1, wherein the C2c1 endonuclease further comprises a nuclear localization signal.

8. The genome editing composition of any one of claims 1 to 7, wherein the genome editing composition is applied to an eukaryotic cell or an eukaryotic organism.

9. A genome editing method, comprising a step of introducing the genome editing composition of any one of claims 1 to 7 into an isolated cell or an organism exclusive of humans.

10. The genome editing method of claim 9, wherein the cell is an eukaryotic cell and the organism is an eukaryotic organism exclusive of humans.

11. An isolated genetically modified cell, having the genome editing composition of any one of claims 1 to 7 introduced thereinto.

12. A genetically modified organism, exclusive of a human, having the genome editing composition of any one of claims 1 to 7 introduced thereinto.
